(19)

Europäisches
Patentamt
European
Patent Office
Office européen
des brevets

(11) **EP 3 344 346 B1**

(12) **EUROPEAN PATENT SPECIFICATION**

(45) Date of publication and mention
of the grant of the patent:
**19.02.2020 Bulletin 2020/08**

(21) Application number: **17735346.3**

(22) Date of filing: **23.06.2017**

(51) Int Cl.:
*A61Q 11/00* (2006.01)      *A61K 8/27* (2006.01)
*A61K 8/44* (2006.01)       *A61K 8/73* (2006.01)
*A61K 8/24* (2006.01)

(86) International application number:
**PCT/US2017/039084**

(87) International publication number:
**WO 2017/223497 (28.12.2017 Gazette 2017/52)**

(54) **ORAL CARE COMPOSITIONS AND METHODS OF USE**

MUNDPFLEGEZUSAMMENSETZUNGEN UND VERFAHREN ZUR VERWENDUNG

COMPOSITIONS DE SOINS BUCCAUX ET PROCÉDÉS D'UTILISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.06.2016 PCT/CN2016/086994**

(43) Date of publication of application:
**11.07.2018 Bulletin 2018/28**

(73) Proprietor: **Colgate-Palmolive Company
New York, NY 10022 (US)**

(72) Inventors:
• **POTH, Tilo**
 **69469 Weinheim (DE)**
• **POTANIN, Andrei**
 **Hillsborough, NJ 08844 (US)**
• **BLANVALET, Claude**
 **B-4031 Angleur (BE)**
• **WON, Betty**
 **Princeton Junction, NJ 08550 (US)**
• **MANUS, Lisa**
 **Lawrenceville, NJ 08648 (US)**
• **STRANICK, Michael A.**
 **Bridgewater, New Jersey 08807 (US)**
• **HUANG, Xiao Yi**
 **Guangzhou City**
 **Guangdong 510730 (CN)**
• **PRENCIPE, Michael**
 **West Windsor, New Jersey 08550 (US)**
• **RUSSO, Amy**
 **Piscataway, NJ 08854 (US)**

• **STETTLER, Hansruedi**
 **CH-4054 Basel (CH)**
• **YAN, Peng**
 **Guangdong,**
 **Guangzhou 510730 (CN)**
• **TAN, Chengkang**
 **Guangzhou City**
 **Guangdong 510730 (CN)**
• **PATEL, Vyoma**
 **Hillsborough, NJ 08844 (US)**
• **MORGAN, Andre Michelle**
 **Robbinsville, New Jersey 08690 (US)**

(74) Representative: **Sonnenhauser, Thomas Martin
Wuesthoff & Wuesthoff
Patentanwälte PartG mbB
Schweigerstraße 2
81541 München (DE)**

(56) References cited:
**WO-A1-2015/094152      WO-A1-2015/094849
JP-A- H07 304 641       JP-A- 2011 105 682
US-A1- 2007 224 134     US-A1- 2010 135 921
US-A1- 2015 320 654**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 344 346 B1

**(Cont. next page)**

• H Hx ET AL: "PRODUCT DATA Ashland Specialty Ingredients Types and Specifications C, mPa.s (Method N5-5) TYPES Viscosity measured at a concentration of Brookfield LVF setting Non-R X W and D R B 1% 2% 5% Spindle no", Ashland. Rev, 1 January 2011 (2011-01-01), pages 5-2015, XP55395554, Retrieved from the Internet: URL:http://www.brenntag.com/media/documents/bsi/product_data_sheets/material_science/ashland_cellulose_rheology_modifiers/natrosol_250_pds.pdf

Remarks:

The file contains technical information submitted after the application was filed and not included in this specification

Remarks:

The file contains technical information submitted after the application was filed and not included in this specification

**Description**

BACKGROUND

Arginine-based oral care compositions generally include some combination of polymers, abrasive(s); and in some instances, additional active ingredients. In those instances where additional active ingredients are included and comprise cationic metal ions, e.g. zinc, maintaining the physical stability of the composition is a challenge because of the interaction between these cationic metal ions and certain polymeric components and abrasive systems.

The use of certain abrasives or specific concentrations of particular polymers are two ways in which the stability issues have been addressed. However, these methods have generally been individually focused on stand-up (i.e., appearance on the brush) and squeezability from packaging (toothpaste tubes). As such, there remains a need to reconcile these physical stability concerns and processability. Certain embodiments of the present invention are designed to address this need.

BRIEF SUMMARY

Some embodiments of the present invention provide oral care compositions comprising a basic amino acid in free or salt wherein the amino acid is selected from arginine, lysine, and a combination thereof; a combination of zinc ion sources; and a thickening system comprising from 0.25 wt.% to 1 wt.% of a nonionic cellulose ether having a molecular weight of from 300,000 to 350,000; and from 0.4 wt.% to 1 wt.% of a polysaccharide gum. In some embodiments, the nonionic cellulose ether is hydroxyethylcellulose and the polysaccharide gum is xanthan gum.

Still further embodiments provide oral care compositions comprising a basic amino acid in free or salt wherein the amino acid is selected from arginine, lysine, and a combination thereof; a combination of zinc ion sources; a thickening system comprising from 0.25 wt.% to 1 wt.% of a nonionic cellulose ether having a molecular weight of from 300,000 to 350,000; and from 0.4 wt.% to 1 wt.% of a polysaccharide gum; and a silica abrasive which exhibits an approximately neutral pH when measured in an aqueous medium.

In some embodiments, the oral care compositions of the present invention demonstrate the ability to avoid viscosity loss and maintain static yield stress over an extended period of time, e.g. after one year.

In another embodiment, the invention encompasses the use of the compositions of the invention in a method to improve oral health comprising applying an effective amount of the oral composition of any of the embodiments set forth above to the oral cavity of a subject in need thereof, e.g.,

> i. a method to reduce or inhibit formation of dental caries,
> ii. reduce or inhibit demineralization of the teeth,
> iii. reduce hypersensitivity of the teeth,
> iv. reduce or inhibit gingivitis,
> v. promote healing of sores or cuts in the mouth,
> vi. reduce levels of acid producing bacteria,
> vii. to increase relative levels of arginolytic bacteria,
> viii. inhibit microbial bio film formation in the oral cavity,
> ix. reduce plaque accumulation,
> x. reduce erosion of the teeth, and/or
> xi. clean the teeth and oral cavity.

Further disclosed is the use of sodium bicarbonate, sodium methyl cocoyl taurate (tauranol), methylisothiazolinone, and benzyl alcohol and combinations thereof in the manufacture of a Composition of the Invention.

DETAILED DESCRIPTION

As used herein, the terms "oral composition" and "oral care composition" mean the total composition that is delivered to the oral surfaces. The composition is further defined as a product which, during the normal course of usage, is not, intended for systemic administration of particular therapeutic agents or intentionally swallowed; but rather, is retained in the oral cavity for a time sufficient to contact substantially all of the dental surfaces and/or oral tissues for the purposes of oral activity. Examples of such compositions include, but are not limited to, toothpaste or a dentifrice, a mouthwash or a mouth rinse, a topical oral gel, a denture cleanser, and the like.

As used herein, the term "dentifrice" means paste, gel, or liquid formulations unless otherwise specified. The dentifrice composition can be in any desired form such as deep striped, surface striped, multi-layered, having the gel surrounding the paste, or any combination thereof. Alternatively the oral composition is provided as a dual phase composition, wherein individual compositions are combined when dispensed from a separated compartment dispenser.

Basic Amino Acids

According to the invention, a basic amino acid in free or salt form is used, wherein the basic amino acid is selected from arginine, lysine, and a combination thereof. A basic amino acids which can additionally be used in the compositions of the invention include not only naturally occurring basic amino acids, such as histidine, but also any basic amino acids having a carboxyl group and an amino group in the molecule, which are water-soluble and provide an aqueous solution with a pH of 7 or greater.

Accordingly, basic amino acids include, but are not limited to, arginine, lysine, serine, citrullene, ornithine, creatine, histidine, diaminobutanoic acid, diaminoproprionic acid, salts thereof or combinations thereof. In a particular embodiment, the basic amino acids are selected from arginine, citrullene, and ornithine.

In certain embodiments, the basic amino acid is arginine, for example, L-arginine, or a salt thereof.

The compositions of the invention are intended for topical use in the mouth and so salts for use in the present invention should be safe for such use, in the amounts and concentrations provided. Suitable salts include salts known in the art to be pharmaceutically acceptable salts which are generally considered to be physiologically acceptable in the amounts and concentrations provided. Physiologically acceptable salts include those derived from pharmaceutically acceptable inorganic or organic acids or bases, for example acid addition salts formed by acids which form a physiological acceptable anion, e.g., hydrochloride or bromide salt, and base addition salts formed by bases which form a physiologically acceptable cation, for example those derived from alkali metals such as potassium and sodium or alkaline earth metals such as calcium and magnesium. Physiologically acceptable salts may be obtained using standard procedures known in the art, for example, by reacting a sufficiently basic compound such as an amine with a suitable acid affording a physiologically acceptable anion.

Fluoride Ion Source

The oral care compositions may further include one or more fluoride ion sources, e.g., soluble fluoride salts. A wide variety of fluoride ion-yielding materials can be employed as sources of soluble fluoride in the present compositions. Examples of suitable fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al.. Representative fluoride ion sources used with the present invention include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In certain embodiments the fluoride ion source includes stannous fluoride, sodium fluoride, sodium monofluorophosphate as well as mixtures thereof. Where the formulation comprises calcium salts, the fluoride salts are preferably salts wherein the fluoride is covalently bound to another atom, e.g., as in sodium monofluorophosphate, rather than merely ionically bound, e.g., as in sodium fluoride.

Surfactants

The invention may in some embodiments contain anionic surfactants, for example, water-soluble salts of higher fatty acid monoglyceride monosulfates, such as the sodium salt of the monosulfated monoglyceride of hydrogenated coconut oil fatty acids such as sodium N- methyl N-cocoyl taurate, sodium coco-glyceride sulfate; higher alkyl sulfates, such as sodium lauryl sulfate; higher alkyl-ether sulfates, e.g., of formula $CH_3(CH_2)_mCH_2(OCH_2CH_2)_nOSO_3X$, wherein m is 6-16, e.g., 10, n is 1-6, e.g., 2, 3 or 4, and X is Na or , for example sodium laureth-2 sulfate $(CH_3(CH2)_{10}CH_2(OCH_2CH_2)_2OSO_3Na)$; higher alkyl aryl sulfonates such as sodium dodecyl benzene sulfonate (sodium lauryl benzene sulfonate); higher alkyl sulfoacetates, such as sodium lauryl sulfoacetate (dodecyl sodium sulfoacetate), higher fatty acid esters of 1,2 dihydroxy propane sulfonate, sulfocolaurate (N-2- ethyl laurate potassium sulfoacetamide) and sodium lauryl sarcosinate. By "higher alkyl" is meant, e.g., $C_{6-3}o$ alkyl. In particular embodiments, the anionic surfactant (where present) is selected from sodium lauryl sulfate and sodium ether lauryl sulfate. When present, the anionic surfactant is present in an amount which is effective, e.g., > 0.001% by weight of the formulation, but not at a concentration which would be irritating to the oral tissue, e.g., 1 %, and optimal concentrations depend on the particular formulation and the particular surfactant. In one embodiment, the anionic surfactant is present at from 0.03% to 5% by weight, e.g., 1.5%.

Cationic surfactants useful in the present invention can be broadly defined as derivatives of aliphatic quaternary ammonium compounds having one long alkyl chain containing 8 to 18 carbon atoms such as lauryl trimethylammonium chloride, cetyl pyridinium chloride, cetyl trimethylammonium bromide, di-isobutylphenoxyethyldimethylbenzylammonium chloride, coconut alkyltrimethylammonium nitrite, cetyl pyridinium fluoride, and mixtures thereof. Illustrative cationic surfactants are the quaternary ammonium fluorides described in U.S. Pat. No. 3,535,421, to Briner et al.. Certain cationic surfactants can also act as germicides in the compositions.

Illustrative nonionic surfactants that can be used in the compositions of the invention can be broadly defined as compounds produced by the condensation of alkylene oxide groups (hydrophilic in nature) with an organic hydrophobic compound which may be aliphatic or alkylaromatic in nature. Examples of suitable nonionic surfactants include, but are not limited to, the Pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials. In a particular embodiment, the composition of the invention comprises a nonionic surfactant selected from polaxamers (e.g., polaxamer 407), polysorbates (e.g., polysorbate 20), polyoxyl hydrogenated castor oils (e.g., polyoxyl 40 hydrogenated castor oil), and mixtures thereof.

Illustrative amphoteric surfactants that can be used in the compositions of the invention include betaines (such as cocamidopropylbetaine), derivatives of aliphatic secondary and tertiary amines in which the aliphatic radical can be a straight or branched chain and wherein one of the aliphatic substituents contains about 8-18 carbon atoms and one contains an anionic water-solubilizing group (such as carboxylate, sulfonate, sulfate, phosphate or phosphonate), and mixtures of such materials.

Illustrative zwitterionic surfactants that can be used in the compositions of the invention include derivatives of aliphatic quaternary ammonium, phosphonium and sulfonium compounds in which the aliphatic radical can be a straight or branched chain and wherein one of the aliphatic substituents contains about 8-18 carbon atoms and one contains an anionic water-solubilizing group (such as carboxy, sulfonate, sulfate, phosphate or phosphonate). The surfactant or mixtures of compatible surfactants can be present in the compositions of the present invention in 0.1% to 5%, in another embodiment 0.3% to 3% and in another embodiment 0.5% to 2% by weight of the total composition.

Flavoring Agents

The oral care compositions of the invention may also include a flavoring agent. Flavoring agents which are used in the practice of the present invention include, but are not limited to, essential oils and various flavoring aldehydes, esters, alcohols, and similar materials, as well as sweeteners such as sodium saccharin. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint.

The flavoring agent is incorporated in the oral composition at a concentration of 0.01 to 1% by weight.

Chelating and anti-calculus agents

The oral care compositions of the invention also may include one or more chelating agents able to complex calcium found in the cell walls of the bacteria. Binding of this calcium weakens the bacterial cell wall and augments bacterial lysis.

Another group of agents suitable for use as chelating or anti-calculus agents in the present invention are the soluble pyrophosphates. The pyrophosphate salts used in the present compositions can be any of the alkali metal pyrophosphate salts. In certain embodiments, salts include tetra alkali metal pyrophosphate, dialkali metal diacid pyrophosphate, trialkali metal monoacid pyrophosphate and mixtures thereof, wherein the alkali metals are sodium or potassium. The salts are useful in both their hydrated and unhydrated forms. An effective amount of pyrophosphate salt useful in the present composition is generally enough to provide least 0.1 wt. % pyrophosphate ions, e.g., 0.1 to 3 wt.%, e.g., 0.1 to 2 wt.%, e.g., 0.1 to 1 wt.%, e.g., 0.2 to 0.5 wt.%. The pyrophosphates also contribute to preservation of the compositions by lowering the effect of water activity.

Polymers

The oral care compositions of the invention also optionally include one or more polymers, such as polyethylene glycols, polyvinyl methyl ether maleic acid copolymers, polysaccharides (e.g., cellulose derivatives, for example carboxymethyl cellulose, or polysaccharide gums, for example xanthan gum or carrageenan gum). Acidic polymers, for example polyacrylate gels, may be provided in the form of their free acids or partially or fully neutralized water soluble alkali metal (e.g., potassium and sodium) or ammonium salts. Certain embodiments include 1 :4 to 4: 1 copolymers of maleic anhydride or acid with another polymerizable ethylenically unsaturated monomer, for example, methyl vinyl ether (methoxyethylene) having a molecular weight (M.W.) of about 30,000 to about 1,000,000. These copolymers are available for example as Gantrez AN 139(M.W. 500,000), AN 1 19 (M.W. 250,000) and S-97 Pharmaceutical Grade (M.W. 70,000), of GAF Chemicals Corporation.

Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1 103, M.W. 10,000 and EMA Grade 61, and 1 : 1 copolymers of acrylic acid with methyl or hydroxyethyl methacrylate, methyl or ethyl acrylate, isobutyl vinyl ether or N-vinyl-2-pyrrolidone.

Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorosorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility.

A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of about 1,000 to about 2,000,000, described in U.S. Pat. No. 4,842,847, Jun. 27, 1989 to Zahid.

Another useful class of polymeric agents includes polyamino acids, particularly those containing proportions of anionic surface-active amino acids such as aspartic acid, glutamic acid and phosphoserine, as disclosed in U.S. Pat. No. 4,866,161 Sikes et al..

In preparing oral care compositions, it is sometimes necessary to add some thickening material to provide a desirable consistency or to stabilize or enhance the performance of the formulation. In certain embodiments, the thickening agents are carboxyvinyl polymers, carrageenan, xanthan gum, hydroxyethyl cellulose and water soluble salts of cellulose ethers such as sodium carboxymethyl cellulose and sodium carboxymethyl hydroxyethyl cellulose. Natural gums such as karaya, gum arabic, and gum tragacanth can also be incorporated. Colloidal magnesium aluminum silicate or finely divided silica can be used as component of the thickening composition to further improve the composition's texture.

## Abrasives

Generally, the inclusion of abrasives in dentifrice formulations is necessary for effective cleaning of teeth by brushing. It has been determined that by including an abrasive silica having an acid pH in the composition, compositions of enhanced viscosity stability are obtained. Prophy silica available from Grace, offered as Sylodent™, can be used with various embodiments of the present invention.

The acidic silica abrasive is included in the dentifrice components at a concentration of about 2 to about 35% by weight; about 3 to about 20 % by weight, about 3 to about 15% by weight, about 10 to about 15 % by weight. For example, the acidic silica abrasive may be present in an amount selected from 2 wt.%, 3 wt.%, 4 wt.%, 5 wt.%, 6 wt.%, 7 wt.%, 8 wt.%, 9 wt.%, 10 wt.%, 11 wt.%, 12 wt.%, 13 wt.%, 14 wt.%, 15 wt.%, 16 wt.%, 17 wt.%, 18 wt.%, 19 wt.%, 20 wt.%.

A commercially available acidic silica abrasive is Sylodent 783 available from W. R. Grace & Company, Baltimore, Md. Sylodent 783 has a pH of 3.4-4.2 when measured as a 5% by weight slurry in water. For use in the present invention, the silica material has an average particle size of less than 10 microns, e.g., 3-7 microns, e.g. about 5.5 microns. For example a small particle silica may have an average particle size (D50) of 2.5 - 4.5 microns.

The composition may also include any silica suitable for oral care compositions, such as precipitated silicas or silica gels. For example synthetic amorphous silica. Silica may also be available as a thickening agent, e.g., particle silica. For example, the silica can also be small particle silica (e.g., Sorbosil AC43 from PQ Corporation, Warrington, United Kingdom). However the additional abrasives are preferably not present in a type or amount so as to increase the RDA of the dentifrice to levels which could damage sensitive teeth, e.g., greater than 130.

The invention may also comprise a commercially available cleaning silica in certain embodiments of the invention. Zeodent 114 offered by J.M. Huber Finland Oy Telakkatie 5 FIN-49460 Hamina, is one such commercially available silica.

## Water

Water is present in the oral compositions of the invention. Water, employed in the preparation of commercial oral compositions should be deionized and free of organic impurities. Water commonly makes up the balance of the compositions and includes 5% to 45%, e.g., 10% to 20%, e.g., 25 - 35%, by weight of the oral compositions. This amount of water includes the free water which is added plus that amount which is introduced with other materials such as with sorbitol or silica or any components of the invention. The Karl Fischer method is a one measure of calculating free water.

Humectants

Within certain embodiments of the oral compositions, it is also desirable to incorporate a humectant to reduce evaporation and also contribute towards preservation by lowering water activity. Certain humectants can also impart desirable sweetness or flavor to the compositions. The humectant, on a pure humectant basis, generally includes 15% to 70% in one embodiment or 30% to 65% in another embodiment by weight of the composition.

Suitable humectants include edible polyhydric alcohols such as glycerin, sorbitol, xylitol, propylene glycol as well as other polyols and mixtures of these humectants. Mixtures of glycerin and sorbitol may be used in certain embodiments as the humectant component of the compositions herein.

The present invention in its method aspect involves applying to the oral cavity a safe and effective amount of the compositions described herein.

The compositions according to the invention can be incorporated into oral compositions for the care of the mouth and teeth such as toothpastes, transparent pastes, gels, mouth rinses, sprays and chewing gum.

In some embodiments, the present invention provides an oral care composition comprising: a basic amino acid in free or salt wherein the amino acid is selected from arginine, lysine, and a combination thereof; a combination of zinc ion sources; and a thickening system comprising: from 0.25 wt.% to 1 wt.% of a nonionic cellulose ether having a molecular weight of from 300,000 to 350,000; and from 0.4 wt.% to 1 wt.% of a polysaccharide gum. In some embodiments, the present invention provides an oral care composition comprising: a basic amino acid in free or salt wherein the amino acid is selected from arginine, lysine, and a combination thereof; a combination of zinc ion sources; and a thickening system comprising: from about 0.5 wt.% to about 1 wt.% of a nonionic cellulose ether having a molecular weight of from 300,000 to 350,000; and from about 0.4 wt.% to about 0.75 wt.% of a polysaccharide gum.

According to the invention, the compositions comprise a nonionic cellulose ether having a molecular weight of 300,000 to 350,000. Embodiments provide compositions comprising a nonionic cellulose ether having a molecular weight of 300,000. While other embodiments provide compositions comprising a nonionic cellulose ether having a molecular weight of 350,000 (e.g. such as Tylose variants available SE Tylose GmbH & Co. KG).

In some embodiments, the nonionic cellulose ether comprises hydroxyethylcellulose. In further embodiments, the oral care composition comprises from about 0.5 wt.% to about 1 wt.% of hydroxyethylcellulose. Still further embodiments provide oral care compositions comprising from about 0.6 wt.% to about 0.8 wt.% of hydroxyethylcellulose. Yet other embodiments provide oral care compositions comprising about 0.5 wt.%, about 0.6 wt.%, about 0.7 wt.%, about 0.8 wt.%, about 0.9 wt.% or about 1 wt.% of hydroxyethylcellulose. Some embodiments provide oral care compositions comprising 0.5 wt.%, 0.6 wt.%, 0.7 wt.%, 0.8 wt.%, 0.9 wt.% or 1 wt.% of hydroxyethylcellulose.

In some embodiments, the hydroxyethylcellulose has a viscosity, measured at 2% in water at 25 °C, of about 150 to about 400 cps. In some embodiments, the hydroxyethylcellulose having a viscosity, measured at 2% in water at 25 °C, of about 150 to about 400 cps at 25 °C is present in an amount of from about 0.5 wt.% to about 1 wt.%. Some embodiments provide oral care compositions comprising 0.5 wt.%, 0.6 wt.%, 0.7 wt.%, 0.8 wt.%, 0.9 wt.% or 1 wt.% of a hydroxyethylcellulose having a viscosity, measured at 2% in water at 25 °C, of about 150 to about 400 cps.

Some embodiments provide oral care compositions comprising hydroxyethylcellulose having a molecular weight of 300,000 or 350,000, in the amount of from about 0.5 wt.% to about 1 wt.%. Other embodiments provide oral care compositions comprising 0.5 wt.%, 0.6 wt.%, 0.7 wt.%, 0.8 wt.%, 0.9 wt.% or 1 wt.% of a hydroxyethylcellulose having a molecular weight of 300,000. Certain embodiments provide oral care compositions comprising 0.5 wt.%, 0.6 wt.%, 0.7 wt.%, 0.8 wt.%, 0.9 wt.% or 1 wt.% of a hydroxyethylcellulose having a molecular weight of 350,000.

In some embodiments, the polysaccharide gum is xanthan gum. In some embodiments, the oral care composition comprises from 0.4 wt.% to 1 wt.% of xanthan gum. In some embodiments, the oral care composition comprises from 0.4 wt.% to 0.9 wt.% of xanthan gum. In some embodiments, the oral care composition comprises from 0.4 wt.% to 0.75 wt.% of xanthan gum. In some embodiments, the oral care composition comprises from 0.4 wt.% to 0.7 wt.% of xanthan gum. In some embodiments, the oral care composition comprises 0.4 wt.%, about 0.5 wt.%, about 0.6 wt.% or about 0.7 wt.% of xanthan gum.

In some embodiments, the present invention provides an oral care composition comprising: a basic amino acid in free or salt wherein the amino acid is selected from arginine, lysine, and a combination thereof; a combination of zinc ion sources; and a thickening system comprising: from about 0.5 wt.% to about 1 wt.% of a nonionic cellulose ether having a molecular weight of from 300,000 to 350,000; and from 0.4 wt.% to 0.75 wt.% of a polysaccharide gum. In some embodiments, the present invention provides an oral care composition comprising: a basic amino acid in free or salt wherein the amino acid is selected from arginine, lysine, and a combination thereof; a combination of zinc ion sources; and a thickening system comprising: from 0.6 wt.% to 0.8 wt.% of a nonionic cellulose ether having a molecular weight of from 300,000 to 350,000; and from 0.7 wt.% to 0.8 wt.% of a polysaccharide gum.

In some embodiments, the thickening system further comprises from 5 wt.% to 10 wt.% silica. In some embodiments, the thickening system comprises from 0.65 wt.% to 15 wt.% of the oral care composition.

In certain embodiments, the hydroxyethylcellulose and the polysaccharide gum are present in a weight ratio of from about 8:1 to about 1:10. In other embodiments, the hydroxyethylcellulose and the polysaccharide gum are present in a weight ratio of from about 4:1 to about 1:2. Yet other embodiments provide compositions wherein the hydroxyethylcellulose and the polysaccharide gum are present in a weight ratio of from about 3:1 to about 1:1.

In some embodiments, the oral care composition further comprises a fluoride ion source selected from sodium fluoride, sodium monofluorophosphate, and stannous fluoride.

In some embodiments, the oral care composition comprises about 1.0 wt.% zinc oxide; about 0.5 wt.% zinc citrate; about 1.5 wt.% L-arginine; from about 0.3 wt.% to about 0.8 wt.% of xanthan gum; and from about 0.5 wt.% to about 1 wt.% of hydroxyethylcellulose. Some embodiments comprise from 0.4 wt.% to 0.6 wt.% of xanthan gum.

In further embodiments, the oral care composition loses no more than about 45% of its initial viscosity after one year. In some embodiments, the oral care composition loses no more than about 40% of its initial viscosity after one year. In other embodiments, the oral care composition loses no more than about 35% of its initial viscosity after one year. In yet other embodiments, the oral care composition loses no more than about 30% of its initial viscosity after one year. In some embodiments, the oral care composition loses no more than about 25%, 24%, 23%, 22%, 21% or 20% of its initial viscosity after one year.

In some embodiments, the oral care composition has a G'/G" ratio of greater than 0.5. In some embodiments, the oral care composition has a G'/G" ratio of greater than 0.75. In some embodiments, the oral care composition has a G'/G" ratio of greater than 1. In some embodiments, the oral care composition has a G'/G" ratio of greater than 1.5. In some embodiments, the oral care composition has a G'/G" ratio of less than 2. In some embodiments, the oral care composition has a G'/G" ratio of less than 1.5. In some embodiments, the oral care composition has a G'/G" ratio of less than 1. Methods of quantifying the elastic modulus (G'), the loss modulus (G") and G'/G" ratios are described, for example, in WO 2013/089734 A1.

In some embodiments, the compositions of the present invention provide a consistency, K, less than 30 $Pa*s^n$ In some embodiments, the compositions of the present invention provide a flow index, n, of greater than 0.3. In some embodiments, the compositions of the present invention provide a consistency, K, less than 30 $Pa*s^n$ and a flow index, n, of greater than 0.3. In some embodiments, the compositions of the present invention provide a consistency, K, less than 30 $Pa*s^n$; a flow index, n, of greater than 0.3; and a G'/G" ratio of less than 2. In some embodiments, the compositions of the present invention provide a flow index, n, of greater than 0.3; and a G'/G" ratio of less than 2. In some embodiments, the compositions of the present invention provide a consistency, K, less than 30 $Pa*s^n$; and a G'/G" ratio of less than 2.

In some embodiments, the oral care compositions of the present invention provide a yield stress greater than 25 Pa. In other embodiments, the oral care compositions of the present invention provide a yield stress greater than 30 Pa. Yet further embodiments, provide oral care compositions that demonstrate a yield stress greater than 35 Pa.

As used throughout, ranges are used as shorthand for describing each and every value that is within the range. Any value within the range can be selected as the terminus of the range. In the event of a conflict in a definition in the present disclosure and that of a cited reference, the present disclosure controls. It is understood that when formulations are described, they may be described in terms of their ingredients, as is common in the art, notwithstanding that these ingredients may react with one another in the actual formulation as it is made, stored and used, and such products are intended to be covered by the formulations described.

The following examples further describe and demonstrate illustrative embodiments within the scope of the present invention. The examples are given solely for illustration and are not to be construed as limitations of this invention as many variations are possible without departing from the spirit and scope thereof.

EXAMPLES

Example 2

Table 5 (below) describes the formulas of three exemplary compositions of the present invention (Compositions 9, 10 and 11) and a comparative example (Comparative Example I).

Table 5

| | Composition 9 | Composition 10 | Composition 11 | | Comparative Example I |
|---|---|---|---|---|---|
| Ingredient | Wt% | | | | |
| GLYCERIN | 35.00000 | 26.00000 | 26.00000 | | 35.00000 |
| SORBITOL - NON - CRYSTAL - 70% SOLN | -- | 13.00000 | 13.00000 | | -- |

(continued)

| | Composition 9 | Composition 10 | Composition 11 | | Comparative Example I |
|---|---|---|---|---|---|
| **Ingredient** | **Wt%** | | | | |
| EP PURIFIED WATER | 30.90474 | 27.82474 | 27.62474 | | 30.84874 |
| STANDARD ABRASIVE SILICA | 10.00000 | 10.00000 | 10.00000 | | 10.00000 |
| SILICA-THICKENER | 7.00000 | 6.00000 | 6.00000 | | 7.00000 |
| SMALL PARTICLE SILICA | 5.00000 | 5.00000 | 5.00000 | | 5.00000 |
| LAURYL SULFATE GRANULES | 2.10526 | 2.10526 | 2.10526 | | 2.10526 |
| Flavor | 1.50000 | 1.50000 | 1.50000 | | 1.50000 |
| L-ARGININE | 1.50000 | 1.50000 | 1.50000 | | 1.50000 |
| COCAMIDOPROPYL BETAINE | 1.25000 | 1.25000 | 1.25000 | | 1.25000 |
| ZINC OXIDE | 1.00000 | 1.00000 | 1.00000 | | 1.00000 |
| TITANIUM DIOXIDE | 0.75000 | 0.75000 | 0.75000 | | -- |
| XANTHAN GUM | 0.60000 | 0.30000** | 0.30000** | | 0.40000 |
| SODIUM CMC - TYPE 12 | -- | -- | -- | | 1.10000 |
| HYDROXYETHYLCELLULOSE (HEC)* | 0.50000 | 0.80000 | 1.00000 | | -- |
| TETRASODIUM PYROPHOSPHATE | 0.50000 | 0.50000 | 0.50000 | | 0.50000 |
| ZINC CITRATE TRIHYDRATE | 0.50000 | 0.50000 | 0.50000 | | 0.50000 |
| POLOXAMER 407 | 0.50000 | 0.50000 | 0.50000 | | 0.50000 |
| BENZYL ALCOHOL | 0.40000 | 0.40000 | 0.40000 | | 0.40000 |
| 85% PHOSPHORIC ACID | 0.35000 | 0.35000 | 0.35000 | | 0.35000 |
| SODIUM FLUORIDE - USP, EP | 0.32000 | 0.32000 | 0.32000 | | 0.32000 |
| Sweeteners | 0.32000 | 0.40000 | 0.40000 | | 0.42000 |
| Additional Colorants | -- | -- | -- | | 0.30600 |
| *HEC having a molecular weight of about 350,000 **not claimed | | | | | |

Example 3

Table 6 (below) describes the results of viscosity and static yield stress evaluations performed on an exemplary composition of the present invention and a reference formula.

Viscosity and Yield Stress are measured on a Brookfield HADV2 viscometer using V74 vane spindle 1.176 cm in length and 0.589 cm in diameter. This viscometer applies a user-controlled angular velocity to the spindle, typically measured in rotations per second (RPM), and reports torque, T%, measured in the percentage of the maximum total torque on the shaft of the spindle. The torque, T, measured in SI units, N*m, is related to T% as reported by the above mentioned viscometer as $T=1.437*10^{-5}*T\%$.

The tests are performed at room temperature (22 to 25 °C). During the test 0.5 RPM of the spindle is first rotated for 400 sec and then RPM is swept from 0.5 to 200 and back to 0.5 in 12 logarithmical steps each way, 10 seconds per step. The viscosity reading is taken at RPM=1 on the decreasing RPM sweep. Viscosity is then calculated from RPM and T as explained in the Brookfield Manual (Operating Instructions) using two conversion parameters SRC (shear rate constant) and SMC (spindle multiplier constant). In this case, the conversion parameters are defined as follows: SMC=290, SRC=0.27.

Static Yield Stress (YS) is calculated as a fitting parameter by fitting experimental T(RPM) dependence on increasing RPM sweep with the theoretical one which was calculated assuming the so-called Casson constitutive equation and is implicitly given by the following equation:

$$RPM = \frac{15}{\pi} \int_{YS}^{SW} \frac{(S^n - YS^n)^{1/n}}{HSV * S} dS$$

where HSV (high-shear viscosity limit) is another fitting parameter, n=0.3, and SW is the stress on the imaginary wall encompassing the vane which is estimated as follows:

$$SW = \frac{2T}{\pi LD^2 (1 + \frac{D}{3L})}$$

T(RPM) is calculated from these two equations numerically. Only data points with RPM from 5 to 200 and T% between 3 and 100 are fitted.

Table 6

| Comparative Example I | | | |
|---|---|---|---|
| Days after Manufacturing | | Viscosity at 1 RPM (cP) | Static Yield Stress (Pa) |
| 1 | | 442984 | 152 |
| 21 | | 277573 | 161 |
| 30 | | 202798 | 133 |
| 37 | | 203931 | 99 |
| 90 | | 167110 | 94 |
| 365 | | 226817 | 40 |
| Composition 11* | | | |
| Days after Manufacturing | | Viscosity at 1 RPM (cP) | Static Yield Stress (Pa) |
| 1 | | 609528 | 161 |
| 14 | | 527956 | 184 |
| 44 | | 425990 | 187 |
| 75 | | 431088 | 199 |
| 290 | | 493401 | 250 |
| *not claimed | | | |

The data described in Table 6 (above) demonstrates the unexpected stabilizing effects provided by the inventive thickening systems of the present invention. Importantly, these effects are observed over an extended period of time, rather than being transient in nature.

Example 4

Table 7 (below) describes the formulas of two additional compositions of the present invention (Compositions 12 and 13) and another comparative formula (Comparative Example II).

Table 7

| Ingredient | Composition 12 | Composition 13 | | Comparative Example II |
|---|---|---|---|---|
| | Wt% | | | |
| GLYCERIN | -- | 26.000000 | | 35.000000 |
| SORBITOL | 39.000000 | 13.000000 | | -- |
| SUCRALOSE | -- | -- | | 0.020000 |
| EP PURIFIED WATER | 27.824737 | 28.244737 | | 30.848737 |
| SODIUM SACCHARIN | 0.400000 | 0.400000 | | 0.400000 |
| STANDARD ABRASIVE SILICA | 10.000000 | 10.000000 | | 10.000000 |
| SILICA-THICKENER | 6.000000 | 6.000000 | | 7.000000 |
| AMORPHOUS SILICA | 5.000000 | 5.000000 | | 5.000000 |
| Na-LAURYL SULFATE GRANULES | 2.105263 | 2.105263 | | 2.105263 |
| Flavor | 1.500000 | 1.500000 | | 1.500000 |
| L-ARGININE | 1.500000 | 1.500000 | | 1.500000 |
| COCAMIDOPROPYL BETAINE | 1.250000 | 1.250000 | | 1.250000 |
| ZINC OXIDE | 1.000000 | 1.000000 | | 1.000000 |
| TITANIUM DIOXIDE | 0.750000 | -- | | 0.300000 |
| XANTHAN GUM | 0.300000** | 0.300000** | | 0.400000 |
| SODIUM CMC - TYPE 12 | -- | -- | | 1.100000 |
| HYDROXYETHYL CELLULOSE (HEC)* | 0.800000 | 1.000000 | | -- |
| TETRASODIUM PYROPHOSPHATE | 0.500000 | 0.500000 | | 0.500000 |
| ZINC CITRATE TRIHYDRATE | 0.500000 | 0.500000 | | 0.500000 |
| POLOXAMER 407 | 0.500000 | 0.500000 | | 0.500000 |
| BENZYL ALCOHOL | 0.400000 | 0.400000 | | 0.400000 |
| 85% PHOSPHORIC ACID | 0.350000 | 0.350000 | | 0.350000 |
| SODIUM FLUORIDE - USP, EP | 0.320000 | 0.320000 | | 0.320000 |
| Additional Colorants | -- | 0.130000 | | 0.006000 |
| *HEC having a molecular weight of about 350,000 | | | | |
| **not claimed | | | | |

Example 5

Table 8 (below) describes the percentage change in viscosity loss and loss of static yield stress for two exemplary compositions of the present invention (Compositions 12 and 13) and a comparative composition (Comparative Example II). Viscosity and Static Yield Stress were calculated in accordance with the methods described in Example 3 herein.

Table 8

| Composition | Viscosity Loss (1 Year) | Loss of Static Yield Stress (After 1 Year) |
|---|---|---|
| Composition 12 | 19 % | Stable |
| Composition 13 | 21 % | Stable |
| Comparative Example II | 49 % | 73 % |

The data described in Table 8 (above) not only shows that compositions of the present invention demonstrate less viscosity loss and static yield stress loss than a comparative composition, but it also demonstrates that the benefits are reproducible.

Example 6

Table 9 (below) describes an exemplary backbone for oral care compositions of the present invention comprising - inter alia - a basic amino acid in free or salt form (e.g. L-arginine); and a combination of zinc ion sources.

Table 9

| | |
|---|---|
| L-Arginine | 1.5 |
| Glycerin | 33.8 |
| Zn Citrate Trihydrate | 0.5 |
| Sodium Fluoride | 0.32 |
| Non-ionic surfactant | 0.5 |
| Alkali Phosphate salt | 0.5 |
| Zinc Oxide | 1 |
| Saccharin | 0.4 |
| Colorant | 0.75 |
| Silica Abrasive(s) | 15 |
| Silica thickener | 7.5 |
| Anionic surfactant | 2 |
| Flavoring agent | 1.5 |
| Preservative | 0.4 |
| Amphoteric surfactant | 1.25 |

Added to this backbone were the various combinations of water, a nonionic cellulose ether (hydroxyethylcellulose [HEC]); and a polysaccharide gum (xanthan gum), described in Table 10 (below).

Table 10

| Composition | Water | Xanthan Gum | HEC** |
|---|---|---|---|
| | Wt.% | | |
| 14 | 32.18 | 0.20* | 0.70 |
| 15 | 32.18 | 0.70 | 0.20* |
| 16 | 32.18 | 0.70 | 0.20* |
| 17 | 32.18 | 0.52 | 0.38 |
| 18 | 32.05 | 0.70 | 0.33 |
| 19 | 31.84 | 0.52 | 0.72 |
| 20 | 31.78 | 0.70 | 0.60 |
| 21 | 31.78 | 0.40 | 0.90 |
| 22 | 31.98 | 0.20* | 0.90 |
| 23 | 31.94 | 0.65 | 0.49 |
| 24 | 32.18 | 0.35* | 0.55 |

(continued)

| Composition | Water | Xanthan Gum | HEC** |
|---|---|---|---|
| | Wt.% | | |
| 25 | 32.02 | 0.48 | 0.57 |
| *not claimed | | | |
| **HEC having a molecular weight of about 300,000 | | | |

Toothpastes were prepared from each of these combinations by first creating a gel comprising water, xanthan gum and hydroxyethylcellulose (HEC); and then combining each gel with the remaining components (see Table 9) in a Ross double planetary mixer. The rheological profiles of both the gel pre-mixes and the toothpaste end products are evaluated.

Example 7

Gels are tested in ARES G2 rheometer by TA Instruments using Couette geometry (standard DIN cell). Two tests are performed: shear rate sweep between 1 and 100 $sec^{-1}$ and frequency sweeps under small-amplitude oscillations. The data of the shear rate sweeps are fitted with power law, i.e.

$$\text{Shear stress} = K * \text{shear rate}^n$$

where consistency, K, and flow index, $n$, are listed in Table 11 (below). Consistency can be as well thought of as viscosity at shear rate 1 $sec^{-1}$. Flow index $n = 1$ corresponds to Newtonian fluid, lower values indicate shear thinning with an extreme case of n = 0 corresponding to yield stress fluid. From frequency sweeps only viscoelastic moduli at 1 Hz are reported below in Table 11, elastic modulus G' and viscous modulus G", as well as their ratio.

Table 11

| Composition | Gel Parameters | | | | |
|---|---|---|---|---|---|
| | K (Pa*s$^n$) | n | G' (Pa) | G" (Pa) | G'/G" |
| 14 | 3.53 | 0.5866 | 7.1 | 8.77 | 0.81 |
| 15 | 20.88 | 0.245 | 48.3 | 18.7 | 2.58 |
| 16 | 23.16 | 0.243 | 45.7 | 17.5 | 2.61 |
| 17 | 13.72 | 0.3298 | 28.3 | 14.6 | 1.94 |
| 18 | 23.59 | 0.269 | 49 | 20.5 | 2.39 |
| 19 | 17.48 | 0.4125 | 32.5 | 23.2 | 1.40 |
| 20 | 22.88 | 0.3409 | 48.6 | 26.9 | 1.81 |
| 21 | 12.29 | 0.4938 | 22.9 | 22.4 | 1.02 |
| 22 | 4.79 | 0.6171 | 8.3 | 12.15 | 0.68 |
| 23 | 20.97 | 0.3278 | 43.3 | 22.4 | 1.93 |
| 24 | 7.11 | 0.4536 | 14.2 | 11.3 | 1.26 |
| 25 | 13.63 | 0.3918 | 26.7 | 17.4 | 1.53 |

Easy processability of the gel requires low viscosity, less shear thinning and less elasticity, which generally correlates with low values of K, G' and G'/G", but higher values of $n$. The exact limits on these parameters depend on the actual design of the mixer.

Example 8

Static yield stress (YS) and viscosity at 0.25 s$^{-1}$ for the finished toothpaste end-products prepared based on the combinations described in Tables 9 and 10, are evaluated. Measurements are performed on ARG2 rheometer by TA Instrument using a cylindrical cup and vane upper geometry. The measurements were performed in the same containers, in which the samples were aged. Those were standard 50 cc centrifuge tubes, available from VWR, into which the samples were placed at the time of preparation. Then the samples were consolidated by centrifuging at low rotations in the centrifuge so as to remove air pockets and aged directly in these tubes at room temperature (RT) or at 49 °C for up to 2 months. Before the measurements the heated tubes were allowed to cool for 2 hours at room temperature and then inserted directly into the cup of the rheometer. To avoid the tubes mobility in the cup, they were wrapped with a thin layer of tape. The vane was inserted into the tubes and samples measured directly inside them. The measurement procedure closely mimicked the one described above for Brookfield viscometer, i.e., a constant shear rate of 0.05 sec$^{-1}$ was applied for 400 sec and followed by shear rate sweeps up and down from 0.1 to 30 sec$^{-1}$. Here shear rate is calculated from angular velocity of the vane, $\Omega$, following TA Instruments conventions as follows:

$$ S = \frac{1+k^2}{1-k^2}\Omega $$

where k is the ratio of the diameter of the vane to the diameter of the tube. Torque, T, on the shaft of the vane was measured. Yield stress was calculated by fitting T($\Omega$) with the theoretical function calculated assuming Casson constitutive equation and implicitly given by the following equation:

$$ \Omega = \frac{1}{2}\int_{SW0}^{SW}\frac{\left(S^n - YS^n\right)^{1/n}}{HSV * S}dS $$

where HSV (high-shear viscosity limit) is another fitting parameter, n=0.2, and SW is the stress on the imaginary wall encompassing the vane which is estimated as follows:

$$ SW = \frac{2T}{\pi LD^2(1+\frac{D}{3L})} $$

and SW0 is the largest of the two values: YS and k$^2$SW. Viscosity, V, at S=0.25 sec$^{-1}$ was calculated according to Casson equation as follows

$$ V = \left(\left(\frac{YS}{S}\right)^n + HSV^n\right)^{1/n} $$

Note that the exponent n used to process these data is different from the one used to process Brookfield data above (0.2 instead of 0.3) to accommodate a wide range of shear rates as measured by a rheometer.

The results of these evaluations are described in Table 12 (below).

Table 12

| Composition | Paste aged at RT for 1 wk | | Paste aged at 49C for 1 month | | Paste aged at RT for 2 month | |
|---|---|---|---|---|---|---|
| | YS (Pa) | Viscosity at 0.25 s$^{-1}$ (cP) | YS (Pa) | Viscosity at 0.25 s$^{-1}$ (cP) | YS (Pa) | Viscosity at 0.25 s$^{-1}$ (cP) |
| 14 | 1.7 | 50,232 | 17.4 | 85,022 | 2.2 | 55,034 |
| 15 | 25.2 | 173,848 | 48.3 | 222,280 | 29.0 | 183,000 |
| 16 | 24.8 | 167,895 | 42.6 | 204,157 | 26.8 | 176,172 |

(continued)

| Composition | Paste aged at RT for 1 wk | | Paste aged at 49C for 1 month | | Paste aged at RT for 2 month | |
|---|---|---|---|---|---|---|
| | YS (Pa) | Viscosity at 0.25 s$^{-1}$ (cP) | YS (Pa) | Viscosity at 0.25 s$^{-1}$ (cP) | YS (Pa) | Viscosity at 0.25 s$^{-1}$ (cP) |
| 17 | 11.9 | 102,691 | 42.6 | 204,157 | 13.4 | 106,199 |
| 18 | 26.0 | 180,787 | 52.1 | 214,586 | 27.6 | 188,500 |
| 19 | 12.1 | 161,077 | 26.0 | 168,495 | 13.4 | 161,060 |
| 20 | 23.4 | 210,000 | 50.5 | 241,336 | 30.7 | 236,770 |
| 21 | 7.7 | 138,867 | 26.0 | 164,016 | 10.8 | 146,088 |
| 22 | 4.6 | 87,257 | 14.9 | 106,338 | 8.4 | 110,207 |
| 23 | 31.2 | 231,467 | 53.7 | 250,236 | 44.7 | 249,000 |
| 24 | 5.8 | 81,449 | 24.2 | 111,160 | N/a | N/a |
| 25 | 9.0 | 120,471 | 34.1 | 155,823 | 13.0 | 133,262 |

The data described in Table 12 (above) demonstrates that exemplary compositions of the present invention will maintain their shape on the brush, while also being easily squeezed from a toothpaste tube.

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material. compositions of the present invention provide a consistency, K, less than 30 Pa*s$^n$ and a flow index, n, of greater than 0.3. In some embodiments, the compositions of the present invention provide a consistency, K, less than 30 Pa*s$^n$; a flow index, n, of greater than 0.3; and a G'/G" ratio of less than 2. In some embodiments, the compositions of the present invention provide a flow index, n, of greater than 0.3; and a G'/G" ratio of less than 2. In some embodiments, the compositions of the present invention provide a consistency, K, less than 30 Pa*s$^n$; and a G'/G" ratio of less than 2.

In some embodiments, the oral care compositions of the present invention provide a yield stress greater than 25 Pa. In other embodiments, the oral care compositions of the present invention provide a yield stress greater than 30 Pa. Yet further embodiments, provide oral care compositions that demonstrate a yield stress greater than 35 Pa.

As used throughout, ranges are used as shorthand for describing each and every value that is within the range. Any value within the range can be selected as the terminus of the range. In addition, all references cited herein are hereby incorporated by reference in their entireties. In the event of a conflict in a definition in the present disclosure and that of a cited reference, the present disclosure controls. It is understood that when formulations are described, they may be described in terms of their ingredients, as is common in the art, notwithstanding that these ingredients may react with one another in the actual formulation as it is made, stored and used, and such products are intended to be covered by the formulations described.

The following examples further describe and demonstrate illustrative embodiments within the scope of the present invention. The examples are given solely for illustration and are not to be construed as limitations of this invention as many variations are possible without departing from the spirit and scope thereof.

EXAMPLES

Example 1

The examples herein detail how the viscosity over time for a composition which exhibits a problem of rapid reduction in viscosity (Run A), is compared to five compositions which show the stabilized viscosity provided by the invention (Compositions 1-5 in Table 1).

Viscosity is measured on a Brookfield HADV2 viscometer using a V74 vane spindle. This viscometer applies a user-controlled angular velocity to the spindle, typically measured in rotations per second (RPM), and reports torque on the shaft of the spindle. Viscosity is then calculated from RPM and torque as explained in the Brookfield Manual (Operating Instructions) using too conversion parameters SRC (shear rate constant) and SMC (spindle multiplier constant). The conversion parameters are defined as follows: SMC=290, SRC=0.2723. The test is performed at room temperature, and varies between 22 and 25 °C. During the test, RPM of the spindle is swept from 200 to 0.5 in 12 steps, 10 seconds per step. The viscosity reading reported is taken at RPM=1.

Compositions containing zinc oxide, zinc citrate, arginine and a fluoride source are prepared as described in Table 1, below. All compositions are formulated to provide a 10% pH of 8 - 8.5 using 0 - 0.35% phosphoric acid. The composition identified as Run A does not contain a silica abrasive which exhibits an acid pH when measured as an aqueous slurry. The compositions identified as Compositions 1-5 in Table 1 (below) contain a silica abrasive which exhibits an acid pH (Prophy Silica - Sylodent 783) when measured as an aqueous slurry in varying amounts, as detailed below.

Table 1. Dentifrice Formulations

| Experiment ID | Run A | Composition 1 | Composition 2 | Composition 3 | Composition 4 | Composition 5 |
|---|---|---|---|---|---|---|
| INGREDIENTS | | | | | | |
| 99.0% - 101.0% GLYCERIN - USP,EP VEG | 35 | 35 | 35 | 35 | 35 | 35 |
| DEMINERALIZED WATER | Q.S. | Q.S. | Q.S. | Q.S. | Q.S. | Q.S. |
| PROPHY SILICA (SYLODENT 783) | 0 | 15 | 10 | 5 | 5 | 3 |
| ABRASIVES (e.g., includes Abrasive silcas, High Cleaning Silicas) | 20 | 5 | 10 | 15 | 15 | 17 |
| SILICA-THICKENER | 6.5 | 7 | 7 | 7 | 7 | 7 |
| ANIONIC SURFACTANT | 2 | 2 | 2 | 2 | 2 | 2 |
| L-ARGININE | 1.5 | 1.5 | 1.5 | 1.5 | 1.5 | 1.5 |
| AMPHOTERIC SURFACTANT | 1.25 | 1.25 | 1.25 | 1.25 | 1.25 | 1.25 |
| NON-IONIC SURFACTANT | 0.75 | 0.75 | 0.75 | 0.75 | 0.75 | 0.75 |
| ZINC OXIDE | 1 | 1 | 1 | 1 | 1 | 1 |
| POLYMER | 1 | 1.3 | 1.3 | 1.3 | 1.3 | 1.3 |
| COLORANT | 0.75 | 0.75 | 0.75 | 0.75 | 0.75 | 0.75 |
| ALKALI PHOSPHATE SALT | 0.5 | 0.5 | 0.5 | 0.5 | 0.5 | 0.5 |
| ZINC CITRATE TRIHYDRATE | 0.5 | 0.5 | 0.5 | 0.5 | 0.5 | 0.5 |
| PRESERVATIVE | 0.4 | 0.4 | 0.4 | 0.4 | 0.4 | 0.4 |
| SODIUM FLUORIDE - USP, EP | 0.32 | 0.32 | 0.32 | 0.32 | 0.32 | 0.32 |
| 85% SYRUPY PHOSPHORIC ACID - FOOD GRADE | 0.35 | 0 | 0 | 0 | 0 | 0 |
| FLAVORING AGENT | 2 | 2 | 2 | 2 | 1.82 | 1.52 |
| TOTAL COMPONENTS | 100 | 100 | 100 | 100 | 100 | 100 |

The composition identified as Run A displays an initial viscosity which is initially 500,000 cps to 600,000 cps high, but decreases to under 400,000 cps in 2 weeks, and under 200,000 cps at 6 weeks. Surprisingly, the compositions containing a silica abrasive which exhibits an acid pH (Prophy Silica - Sylodent 783) when measured as an aqueous slurry, Compositions 1 to 5 in Table 1 (above), eliminate this undesirable characteristic and instead produce viscosities that are stable or increase over time (See, Table 2 below).

Table 2. Viscosity data

| Experiment ID | Run A | Composition 1 | Composition 2 | Composition 3 | Composition 4 | Composition 5 |
|---|---|---|---|---|---|---|
| Time | Viscosity (cps) | | | | | |
| 0 | | | | | 491040 | 363489 |
| 1 d | 539119 | 211912 | 300155 | 272475 | | |
| 5 d | 601597 | | | 309816 | | |
| 1 wk | 627362 | | 288561 | | 383245 | 371651 |
| 2 wk | 433485 | 340733 | | 328495 | 403212 | 364565 |
| 3 wk | | 343310 | 314325 | 334292 | | |
| 4 wk | 224794 | 395483 | 304019 | | 430909 | 423823 |
| 5 wk | | 375515 | 338801 | 322698 | | |
| 6 wk | 193233 | 376804 | 344598 | 334292 | 406432 | 442503 |
| 7 wk | | | 334292 | | | |
| 9 wk | | | 387753 | | | |
| 10 wk | | 351039 | | 364565 | | |
| 11 wk | 158451 | | 373583 | 381956 | | |
| 12 wk | | 405788 | 357480 | | | |
| 13 wk | | 405788 | 398059 | 393550 | | |

Upon further investigation, it was found that the silica abrasive which exhibits an acid pH when measured as an aqueous slurry silica is acidic (pH 3.4 - 4.2) does not require phosphoric acid to adjust the product pH. Other abrasive silicas and high cleaning silicas are about neutral in pH (pH 7 - 8) and thus, require phosphoric acid for pH adjustment.

Table 3

| | Composition 6 | Composition 7 | Composition 8 |
|---|---|---|---|
| Demineralized Water | Q.S. | Q.S. | Q.S. |
| Glycerin - 99.5% | 35 | 35 | 35 |
| Polymer | 1.2 | 1.2 | 1.2 |
| Zinc Oxide | 1 | 1 | 1 |
| Zinc Citrate | 0.5 | 0.5 | 0.5 |
| Alkali Phosphate Salt | 0.5 | 0.5 | 0.5 |
| Flavoring agent | 1.82 | 1.82 | 1.82 |
| Sodium Fluoride | 0.32 | 0.32 | 0.32 |
| Colorant | 0.75 | 0.75 | 0.75 |
| L-Arginine | 1.5 | 1.5 | 1.5 |
| Non-Ionic Surfactant | 0.5 | 0.5 | 0.5 |
| Abrasives (e.g., includes Abrasive Silcas, High Cleaning Silicas) | 8 | 10 | 12 |

(continued)

|  | Composition 6 | Composition 7 | Composition 8 |
|---|---|---|---|
| Prophy silica (Sylodent 783) | 7 | 5 | 3 |
| Silica - thickener | 7 | 7 | 8.5 |
| Preservative | 0.4 | 0.4 | 0.4 |
| Anionic Surfactant | 5.7 | 5.7 | 5.7 |
| Amphoteric Surfactant | 1.25 | 1.25 | 1.25 |
| Total Components | 100 | 100 | 100 |

Upon further investigation, when phosphoric acid is removed from further formulations (Compositions 6-8 in Table 3, above), they demonstrate improvement in viscosity stability, and this viscosity trend remained relatively stable from day 1 to 4 weeks when tested at: room temperature, 40 °C and 49 °C. The data is further detailed in Table 4 below.

Table 4

|  | Composition 6 | | | Composition 7 | | | Composition 8 | | |
|---|---|---|---|---|---|---|---|---|---|
|  | Viscosity, $10^3$ cps | | | Viscosity, $10^3$ cps | | | Viscosity, $10^3$ cps | | |
|  | RT | 40 °C | 49 °C | RT | 40 °C | 49 °C | RT | 40 °C | 49 °C |
| 0 | 471 |  |  | 324 |  |  | 336 |  |  |
| 0.14 | 370 |  |  | 197 |  |  | 268 |  |  |
| 1 | 363 | 418 | 390 | 200 | 227 | 258 | 230 | 256 | 263 |
| 2 | 360 | 430 | 440 | 201 | 243 | 269 | 220 | 250 | 258 |
| 3 | 325 |  |  | 205 | 246 | 245 | 228 | 277 | 274 |
| 4 | 314 | 412 | 385 | 224 | 253 | 253 | 227 | 268 | 272 |
| 6 | 327 |  |  | 218 |  |  | 233 |  |  |

Example 2

Table 5 (below) describes the formulas of three exemplary compositions of the present invention (Compositions 9, 10 and 11) and a comparative example (Comparative Example I).

Table 5

|  | Composition 9 | Composition 10 | Composition 11 |  | Comparative Example I |
|---|---|---|---|---|---|
| Ingredient | Wt% | | | | |
| GLYCERIN | 35.00000 | 26.00000 | 26.00000 |  | 35.00000 |
| SORBITOL - NON-CRYSTAL - 70% SOLN | -- | 13.00000 | 13.00000 |  | -- |
| EP PURIFIED WATER | 30.90474 | 27.82474 | 27.62474 |  | 30.84874 |
| STANDARD ABRASIVE SILICA | 10.00000 | 10.00000 | 10.00000 |  | 10.00000 |
| SILICA-THICKENER | 7.00000 | 6.00000 | 6.00000 |  | 7.00000 |
| SMALL PARTICLE SILICA | 5.00000 | 5.00000 | 5.00000 |  | 5.00000 |
| LAURYL SULFATE GRANULES | 2.10526 | 2.10526 | 2.10526 |  | 2.10526 |
| Flavor | 1.50000 | 1.50000 | 1.50000 |  | 1.50000 |
| L-ARGININE | 1.50000 | 1.50000 | 1.50000 |  | 1.50000 |

(continued)

| Ingredient | Composition 9 | Composition 10 | Composition 11 | | Comparative Example I |
|---|---|---|---|---|---|
| | Wt% | | | | |
| COCAMIDOPROPYL BETAINE | 1.25000 | 1.25000 | 1.25000 | | 1.25000 |
| ZINC OXIDE | 1.00000 | 1.00000 | 1.00000 | | 1.00000 |
| TITANIUM DIOXIDE | 0.75000 | 0.75000 | 0.75000 | | -- |
| XANTHAN GUM | 0.60000 | 0.30000 | 0.30000 | | 0.40000 |
| SODIUM CMC - TYPE 12 | -- | -- | -- | | 1.10000 |
| HYDROXYETHYL-CELLULOSE (HEC)* | 0.50000 | 0.80000 | 1.00000 | | -- |
| TETRASODIUM PYROPHOSPHATE | 0.50000 | 0.50000 | 0.50000 | | 0.50000 |
| ZINC CITRATE TRIHYDRATE | 0.50000 | 0.50000 | 0.50000 | | 0.50000 |
| POLOXAMER 407 | 0.50000 | 0.50000 | 0.50000 | | 0.50000 |
| BENZYL ALCOHOL | 0.40000 | 0.40000 | 0.40000 | | 0.40000 |
| 85% PHOSPHORIC ACID | 0.35000 | 0.35000 | 0.35000 | | 0.35000 |
| SODIUM FLUORIDE - USP, EP | 0.32000 | 0.32000 | 0.32000 | | 0.32000 |
| Sweeteners | 0.32000 | 0.40000 | 0.40000 | | 0.42000 |
| Additional Colorants | -- | -- | -- | | 0.30600 |
| *HEC having a molecular weight of about 350,000 | | | | | |

Example 3

Table 6 (below) describes the results of viscosity and static yield stress evaluations performed on an exemplary composition of the present invention and a reference formula.

Viscosity and Yield Stress are measured on a Brookfield HADV2 viscometer using V74 vane spindle 1.176 cm in length and 0.589 cm in diameter. This viscometer applies a user-controlled angular velocity to the spindle, typically measured in rotations per second (RPM), and reports torque, T%, measured in the percentage of the maximum total torque on the shaft of the spindle. The torque, T, measured in SI units, N*m, is related to T% as reported by the above mentioned viscometer as $T=1.437*10^{-5}*T\%$.

The tests are performed at room temperature (22 to 25 °C). During the test 0.5 RPM of the spindle is first rotated for 400 sec and then RPM is swept from 0.5 to 200 and back to 0.5 in 12 logarithmical steps each way, 10 seconds per step. The viscosity reading is taken at RPM=1 on the decreasing RPM sweep. Viscosity is then calculated from RPM and T as explained in the Brookfield Manual (Operating Instructions) using two conversion parameters SRC (shear rate constant) and SMC (spindle multiplier constant). In this case, the conversion parameters are defined as follows: SMC=290, SRC=0.27.

Static Yield Stress (YS) is calculated as a fitting parameter by fitting experimental T(RPM) dependence on increasing RPM sweep with the theoretical one which was calculated assuming the so-called Casson constitutive equation and is implicitly given by the following equation:

$$RPM = \frac{15}{\pi} \int_{YS}^{SW} \frac{\left(S^n - YS^n\right)^{1/n}}{HSV * S} dS$$

where HSV (high-shear viscosity limit) is another fitting parameter, n=0.3, and SW is the stress on the imaginary wall encompassing the vane which is estimated as follows:

$$SW = \frac{2T}{\pi LD^2(1+\dfrac{D}{3L})}$$

T(RPM) is calculated from these two equations numerically. Only data points with RPM from 5 to 200 and T% between 3 and 100 are fitted.

Table 6

| Comparative Example I | | | |
|---|---|---|---|
| Days after Manufacturing | | Viscosity at 1 RPM (cP) | Static Yield Stress (Pa) |
| 1 | | 442984 | 152 |
| 21 | | 277573 | 161 |
| 30 | | 202798 | 133 |
| 37 | | 203931 | 99 |
| 90 | | 167110 | 94 |
| 365 | | 226817 | 40 |
| Composition 11 | | | |
| Days after Manufacturing | | Viscosity at 1 RPM (cP) | Static Yield Stress (Pa) |
| 1 | | 609528 | 161 |
| 14 | | 527956 | 184 |
| 44 | | 425990 | 187 |
| 75 | | 431088 | 199 |
| 290 | | 493401 | 250 |

The data described in Table 6 (above) demonstrates the unexpected stabilizing effects provided by the inventive thickening systems of the present invention. Importantly, these effects are observed over an extended period of time, rather than being transient in nature.

Example 4

Table 7 (below) describes the formulas of two additional compositions of the present invention (Compositions 12 and 13) and another comparative formula (Comparative Example II).

Table 7

| | Composition 12 | Composition 13 | | Comparative Example II |
|---|---|---|---|---|
| Ingredient | Wt% | | | |
| GLYCERIN | -- | 26.000000 | | 35.000000 |
| SORBITOL | 39.000000 | 13.000000 | | -- |
| SUCRALOSE | -- | -- | | 0.020000 |
| EP PURIFIED WATER | 27.824737 | 28.244737 | | 30.848737 |
| SODIUM SACCHARIN | 0.400000 | 0.400000 | | 0.400000 |
| STANDARD ABRASIVE SILICA | 10.000000 | 10.000000 | | 10.000000 |
| SILICA-THICKENER | 6.000000 | 6.000000 | | 7.000000 |
| AMORPHOUS SILICA | 5.000000 | 5.000000 | | 5.000000 |
| Na-LAURYL SULFATE GRANULES | 2.105263 | 2.105263 | | 2.105263 |

(continued)

| Ingredient | Composition 12 | Composition 13 | | Comparative Example II |
|---|---|---|---|---|
| | Wt% | | | |
| Flavor | 1.500000 | 1.500000 | | 1.500000 |
| L-ARGININE | 1.500000 | 1.500000 | | 1.500000 |
| COCAMIDOPROPYL BETAINE | 1.250000 | 1.250000 | | 1.250000 |
| ZINC OXIDE | 1.000000 | 1.000000 | | 1.000000 |
| TITANIUM DIOXIDE | 0.750000 | -- | | 0.300000 |
| XANTHAN GUM | 0.300000 | 0.300000 | | 0.400000 |
| SODIUM CMC - TYPE 12 | -- | -- | | 1.100000 |
| HYDROXYETHYL CELLULOSE (HEC)* | 0.800000 | 1.000000 | | -- |
| TETRASODIUM PYROPHOSPHATE | 0.500000 | 0.500000 | | 0.500000 |
| ZINC CITRATE TRIHYDRATE | 0.500000 | 0.500000 | | 0.500000 |
| POLOXAMER 407 | 0.500000 | 0.500000 | | 0.500000 |
| BENZYL ALCOHOL | 0.400000 | 0.400000 | | 0.400000 |
| 85% PHOSPHORIC ACID | 0.350000 | 0.350000 | | 0.350000 |
| SODIUM FLUORIDE - USP, EP | 0.320000 | 0.320000 | | 0.320000 |
| Additional Colorants | -- | 0.130000 | | 0.006000 |
| *HEC having a molecular weight of about 350,000 | | | | |

Example 5

Table 8 (below) describes the percentage change in viscosity loss and loss of static yield stress for two exemplary compositions of the present invention (Compositions 12 and 13) and a comparative composition (Comparative Example II). Viscosity and Static Yield Stress were calculated in accordance with the methods described in Example 3 herein.

Table 8

| Composition | Viscosity Loss (1 Year) | Loss of Static Yield Stress (After 1 Year) |
|---|---|---|
| Composition 12 | 19 % | Stable |
| Composition 13 | 21 % | Stable |
| Comparative Example II | 49 % | 73 % |

The data described in Table 8 (above) not only shows that compositions of the present invention demonstrate less viscosity loss and static yield stress loss than a comparative composition, but it also demonstrates that the benefits are reproducible.

Example 6

Table 9 (below) describes an exemplary backbone for oral care compositions of the present invention comprising - inter alia - a basic amino acid in free or salt form (e.g. L-arginine); and a combination of zinc ion sources.

Table 9

| L-Arginine | 1.5 |
|---|---|
| Glycerin | 33.8 |
| Zn Citrate Trihydrate | 0.5 |

(continued)

| | |
|---|---|
| Sodium Fluoride | 0.32 |
| Non-ionic surfactant | 0.5 |
| Alkali Phosphate salt | 0.5 |
| Zinc Oxide | 1 |
| Saccharin | 0.4 |
| Colorant | 0.75 |
| Silica Abrasive(s) | 15 |
| Silica thickener | 7.5 |
| Anionic surfactant | 2 |
| Flavoring agent | 1.5 |
| Preservative | 0.4 |
| Amphoteric surfactant | 1.25 |

Added to this backbone were the various combinations of water, a nonionic cellulose ether (hydroxyethylcellulose [HEC]); and a polysaccharide gum (xanthan gum), described in Table 10 (below).

Table 10

| Composition | Water | Xanthan Gum | HEC** |
|---|---|---|---|
| | Wt.% | | |
| 14 | 32.18 | 0.20 | 0.70 |
| 15 | 32.18 | 0.70 | 0.20 |
| 16 | 32.18 | 0.70 | 0.20 |
| 17 | 32.18 | 0.52 | 0.38 |
| 18 | 32.05 | 0.70 | 0.33 |
| 19 | 31.84 | 0.52 | 0.72 |
| 20 | 31.78 | 0.70 | 0.60 |
| 21 | 31.78 | 0.40 | 0.90 |
| 22 | 31.98 | 0.20 | 0.90 |
| 23 | 31.94 | 0.65 | 0.49 |
| 24 | 32.18 | 0.35 | 0.55 |
| 25 | 32.02 | 0.48 | 0.57 |
| **HEC having a molecular weight of about 300,000 | | | |

Toothpastes were prepared from each of these combinations by first creating a gel comprising water, xanthan gum and hydroxyethylcellulose (HEC); and then combining each gel with the remaining components (see Table 9) in a Ross double planetary mixer. The rheological profiles of both the gel pre-mixes and the toothpaste end products are evaluated.

Example 7

Gels are tested in ARES G2 rheometer by TA Instruments using Couette geometry (standard DIN cell). Two tests are performed: shear rate sweep between 1 and 100 sec$^{-1}$ and frequency sweeps under small-amplitude oscillations. The data of the shear rate sweeps are fitted with power law, i.e.

$$\text{Shear stress} = K * \text{shear rate}^n$$

where consistency, K, and flow index, $n$, are listed in Table 11 (below). Consistency can be as well thought of as viscosity at shear rate 1 sec$^{-1}$. Flow index $n = 1$ corresponds to Newtonian fluid, lower values indicate shear thinning with an extreme case of n = 0 corresponding to yield stress fluid. From frequency sweeps only viscoelastic moduli at 1 Hz are reported below in Table 11, elastic modulus G' and viscous modulus G", as well as their ratio.

Table 11

| Composition | Gel Parameters | | | | |
|---|---|---|---|---|---|
| | K (Pa*s$^n$) | n | G' (Pa) | G" (Pa) | G'/G" |
| 14 | 3.53 | 0.5866 | 7.1 | 8.77 | 0.81 |
| 15 | 20.88 | 0.245 | 48.3 | 18.7 | 2.58 |
| 16 | 23.16 | 0.243 | 45.7 | 17.5 | 2.61 |
| 17 | 13.72 | 0.3298 | 28.3 | 14.6 | 1.94 |
| 18 | 23.59 | 0.269 | 49 | 20.5 | 2.39 |
| 19 | 17.48 | 0.4125 | 32.5 | 23.2 | 1.40 |
| 20 | 22.88 | 0.3409 | 48.6 | 26.9 | 1.81 |
| 21 | 12.29 | 0.4938 | 22.9 | 22.4 | 1.02 |
| 22 | 4.79 | 0.6171 | 8.3 | 12.15 | 0.68 |
| 23 | 20.97 | 0.3278 | 43.3 | 22.4 | 1.93 |
| 24 | 7.11 | 0.4536 | 14.2 | 11.3 | 1.26 |
| 25 | 13.63 | 0.3918 | 26.7 | 17.4 | 1.53 |

Easy processability of the gel requires low viscosity, less shear thinning and less elasticity, which generally correlates with low values of K, G' and G'/G", but higher values of $n$. The exact limits on these parameters depend on the actual design of the mixer.

Example 8

Static yield stress (YS) and viscosity at 0.25 s$^{-1}$ for the finished toothpaste end-products prepared based on the combinations described in Tables 9 and 10, are evaluated. Measurements are performed on ARG2 rheometer by TA Instrument using a cylindrical cup and vane upper geometry. The measurements were performed in the same containers, in which the samples were aged. Those were standard 50 cc centrifuge tubes, available from VWR, into which the samples were placed at the time of preparation. Then the samples were consolidated by centrifuging at low rotations in the centrifuge so as to remove air pockets and aged directly in these tubes at room temperature (RT) or at 49 °C for up to 2 months. Before the measurements the heated tubes were allowed to cool for 2 hours at room temperature and then inserted directly into the cup of the rheometer. To avoid the tubes mobility in the cup, they were wrapped with a thin layer of tape. The vane was inserted into the tubes and samples measured directly inside them. The measurement procedure closely mimicked the one described above for Brookfield viscometer, i.e., a constant shear rate of 0.05 sec$^{-1}$ was applied for 400 sec and followed by shear rate sweeps up and down from 0.1 to 30 sec$^{-1}$. Here shear rate is calculated from angular velocity of the vane, $\Omega$, following TA Instruments conventions as follows:

$$S = \frac{1 + k^2}{1 - k^2} \Omega$$

where k is the ratio of the diameter of the vane to the diameter of the tube. Torque, T, on the shaft of the vane was measured. Yield stress was calculated by fitting $T(\Omega)$ with the theoretical function calculated assuming Casson constitutive equation and implicitly given by the following equation:

$$\Omega = \frac{1}{2} \int_{SW0}^{SW} \frac{\left(S^n - YS^n\right)^{1/n}}{HSV * S} dS$$

where HSV (high-shear viscosity limit) is another fitting parameter, n=0.2, and SW is the stress on the imaginary wall encompassing the vane which is estimated as follows:

$$SW = \frac{2T}{\pi LD^2 (1 + \frac{D}{3L})}$$

and SW0 is the largest of the two values: YS and $k^2$SW. Viscosity, V, at S=0.25 sec$^{-1}$ was calculated according to Casson equation as follows

$$V = \left( \left(\frac{YS}{S}\right)^n + HSV^n \right)^{1/n}$$

Note that the exponent n used to process these data is different from the one used to process Brookfield data above (0.2 instead of 0.3) to accommodate a wide range of shear rates as measured by a rheometer.

The results of these evaluations are described in Table 12 (below).

Table 12

| Composition | Paste aged at RT for 1 wk | | Paste aged at 49C for 1 month | | Paste aged at RT for 2 month | |
|---|---|---|---|---|---|---|
| | YS (Pa) | Viscosity at 0.25 s$^{-1}$ (cP) | YS (Pa) | Viscosity at 0.25 s$^{-1}$ (cP) | YS (Pa) | Viscosity at 0.25 s$^{-1}$ (cP) |
| 14 | 1.7 | 50,232 | 17.4 | 85,022 | 2.2 | 55,034 |
| 15 | 25.2 | 173,848 | 48.3 | 222,280 | 29.0 | 183,000 |
| 16 | 24.8 | 167,895 | 42.6 | 204,157 | 26.8 | 176,172 |
| 17 | 11.9 | 102,691 | 42.6 | 204,157 | 13.4 | 106,199 |
| 18 | 26.0 | 180,787 | 52.1 | 214,586 | 27.6 | 188,500 |
| 19 | 12.1 | 161,077 | 26.0 | 168,495 | 13.4 | 161,060 |
| 20 | 23.4 | 210,000 | 50.5 | 241,336 | 30.7 | 236,770 |
| 21 | 7.7 | 138,867 | 26.0 | 164,016 | 10.8 | 146,088 |
| 22 | 4.6 | 87,257 | 14.9 | 106,338 | 8.4 | 110,207 |
| 23 | 31.2 | 231,467 | 53.7 | 250,236 | 44.7 | 249,000 |
| 24 | 5.8 | 81,449 | 24.2 | 111,160 | N/a | N/a |
| 25 | 9.0 | 120,471 | 34.1 | 155,823 | 13.0 | 133,262 |

The data described in Table 12 (above) demonstrates that exemplary compositions of the present invention will maintain their shape on the brush, while also being easily squeezed from a toothpaste tube.

As used throughout, ranges are used as shorthand for describing each and every value that is within the range. Any value within the range can be selected as the terminus of the range. In addition, all references cited herein are hereby incorporated by referenced in their entireties. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

While the present invention has been described with reference to embodiments, it will be understood by those skilled in the art that various modifications and variations may be made therein without departing from the scope of the present invention as defined by the appended claims.

**Claims**

1. An oral care composition comprising:

    a. a basic amino acid in free or salt form wherein the basic amino acid is selected from arginine, lysine, and a combination thereof;
    b. a combination of zinc ion sources; and
    c. a thickening system comprising:

        i. from 0.25 wt.% to 1 wt.% of a nonionic cellulose ether having a molecular weight of from 300,000 to 350,000; and
        ii. from 0.4 wt.% to 1 wt.% of a polysaccharide gum.

2. The oral care composition according to claim 1, wherein the nonionic cellulose ether has a viscosity, measured at 2% in water at 25 °C, of from 150 to 400 cps.

3. The oral care composition according to any foregoing claim, comprising nonionic cellulose ether in an amount selected from 0.5 wt.% to 1 wt.%, 0.6 wt.% to 0.9 wt.%, 0.5 wt.%, 0.6 wt.%, 0.7 wt.%, 0.8 wt.%, 0.9 wt.% or 1 wt.%.

4. The oral care composition according to any foregoing claim, wherein the nonionic cellulose ether comprises hydroxyethylcellulose.

5. The oral care composition according to any foregoing claim, comprising polysaccharide gum in an amount selected from 0.4 wt.% to 0.9 wt.%, 0.5 wt.% to 0.9 wt.%, from 0.6 wt.% to 0.9 wt.%, about 0.3 wt.%, about 0.4 wt.%, about 0.5 wt.%, about 0.6 wt.%, about 0.7 wt.%, about 0.8 wt.%, or about 0.9 wt.%.

6. The oral care composition according to any foregoing claim, wherein the polysaccharide gum is xanthan gum.

7. The oral care composition according to any foregoing claim, wherein the thickening system further comprises about 5 wt.% to 10 wt.% silica; and/or wherein the nonionic cellulose ether and the polysaccharide gum are present in a weight ratio selected from 3:1 to 1:2, 2:1 to 1:1, about 1:1, 3:1, 2.5:1, 2:1, 1.5:1 or 1:1.

8. The oral care composition according to any foregoing claim, wherein

    (i) the combination of zinc ion sources comprises zinc oxide and zinc citrate, and/or
    (ii) the amino acid is arginine, and is present at about 1.5 wt.%, 5 wt.%, or about 8 wt.%, of the oral care composition, preferably wherein the weight ratio of zinc oxide to zinc citrate is about 2:1.

9. The oral care composition according to claim 8, wherein the zinc citrate is in an amount of about 0.5 wt.% and zinc oxide is present in an amount of about 1.0 wt.% based on the total weight of the oral care composition.

10. The oral care composition according to any foregoing claim, further comprising a fluoride ion source selected from sodium fluoride, sodium monofluorophosphate, and stannous fluoride, preferably wherein the fluoride ion source provides soluble fluoride in an amount of about 1450 ppm, and/or wherein the fluoride ion source comprises stannous fluoride.

11. The oral care composition according to any foregoing claim, comprising:

    a. about 1.0 wt.% zinc oxide;
    b. about 0.5 wt.% zinc citrate;
    c. about 1.5 wt.% L-arginine;
    d. from 0.5 wt.% to 1 wt.% of hydroxyethylcellulose; and

    from 0.3 wt.% to 0.8 wt.% of xanthan gum, preferably in an amount from 0.3 wt.% to 0.6 wt.%, or from 0.7 wt.% to 0.8 wt.%.

12. The oral care composition according to any foregoing claim, wherein the oral care composition

   i) is in a form selected from: a toothpaste, a mouthwash, and a gel; and/or
   ii) has a static yield stress greater than 20 Pa, 25 Pa, 30 Pa, 35 Pa, or 40 Pa.

13. Oral care composition according to any foregoing claim for use in a method to improve oral health comprising applying an effective amount of the said oral care composition to the oral cavity of a subject in need thereof, wherein the method is effective to:

   i. reduce or inhibit formation of dental caries,
   ii. reduce or inhibit demineralization of the teeth,
   iii. reduce hypersensitivity of the teeth,
   iv. reduce or inhibit gingivitis,
   v. promote healing of sores or cuts in the mouth,
   vi. reduce levels of acid producing bacteria,
   vii. to increase relative levels of arginolytic bacteria,
   viii. inhibit microbial bio film formation in the oral cavity,
   ix. reduce plaque accumulation,
   x. reduce erosion of the teeth, and/or
   xi. clean the teeth and oral cavity.

**Patentansprüche**

1. Mundpflegezusammensetzung, umfassend:

   a. eine basische Aminosäure in freier oder Salzform, wobei die basische Aminosäure aus Arginin, Lysin und einer Kombination davon ausgewählt ist;
   b. eine Kombination von Zinkionenquellen; und
   c. ein Verdickungssystem, umfassend:

      i. 0,25 Gew.-% bis 1 Gew.-% eines nichtionischen Celluloseethers mit einem Molekulargewicht von 300.000 bis 350.000; und
      ii. 0,4 Gew.-% bis 1 Gew.-% eines Polysaccharidgummis.

2. Mundpflegezusammensetzung nach Anspruch 1, wobei der nichtionische Celluloseether eine Viskosität, gemessen bei 2 % in Wasser bei 25 °C, von 150 bis 400 cps aufweist.

3. Mundpflegezusammensetzung nach einem vorhergehenden Anspruch, umfassend nichtionischen Celluloseether in einer Menge, ausgewählt aus 0,5 Gew.-% bis 1 Gew.-%, 0,6 Gew.-% bis 0,9 Gew.-%, 0,5 Gew.-%, 0,6 Gew.-%, 0,7 Gew.-%, 0,8 Gew.-%, 0,9 Gew.-% oder 1 Gew.-%.

4. Mundpflegezusammensetzung nach einem vorhergehenden Anspruch, wobei der nichtionische Celluloseether Hydroxyethylcellulose umfasst.

5. Mundpflegezusammensetzung nach einem vorhergehenden Anspruch, umfassend ein Polysaccharidgummi in einer Menge, ausgewählt aus 0,4 Gew.-% bis 0,9 Gew.-%, 0,5 Gew.-% bis 0,9 Gew.-%, 0,6 Gew.-% bis 0,9 Gew.-%, etwa 0,3 Gew.-%, etwa 0,4 Gew.-%, etwa 0,5 Gew.-%, etwa 0,6 Gew.-%, etwa 0,7 Gew.-%, etwa 0,8 Gew.-% oder etwa 0,9 Gew.-%.

6. Mundpflegezusammensetzung nach einem vorhergehenden Anspruch, wobei das Polysaccharidgummi Xanthan ist.

7. Mundpflegezusammensetzung nach einem vorhergehenden Anspruch, wobei das Verdickungssystem des Weiteren etwa 5 Gew.-% bis 10 Gew.-% Siliciumdioxid umfasst; und/oder wobei der nichtionische Celluloseether und das Polysaccharidgummi in einem Gewichtsverhältnis vorliegen, das aus 3:1 bis 1:2, 2:1 bis 1:1, etwa 1:1, 3:1, 2,5:1, 2:1, 1,5:1 oder 1:1 ausgewählt ist.

**EP 3 344 346 B1**

8. Mundpflegezusammensetzung nach einem vorhergehenden Anspruch, wobei

> i) die Kombination von Zinkionenquellen Zinkoxid und Zinkcitrat umfasst, und/oder
> ii) die Aminosäure Arginin ist und mit etwa 1,5 Gew.-%, 5 Gew.-% oder etwa 8 Gew.-% der Mundpflegezusammensetzung vorhanden ist, vorzugsweise wobei das Gewichtsverhältnis von Zinkoxid zu Zinkcitrat etwa 2:1 beträgt.

9. Mundpflegezusammensetzung nach Anspruch 8, wobei das Zinkcitrat in einer Menge von etwa 0,5 Gew.-% und Zinkoxid in einer Menge von etwa 1,0 Gew.-%, bezogen auf das Gesamtgewicht der Mundpflegezusammensetzung, vorhanden ist.

10. Mundpflegezusammensetzung nach einem vorhergehenden Anspruch, des Weiteren umfassend eine Fluoridionenquelle, ausgewählt aus Natriumfluorid, Natriummonofluorphosphat und Zinnfluorid, vorzugsweise wobei die Fluoridionenquelle lösliches Fluorid in einer Menge von etwa 1450 ppm bereitstellt, und/oder wobei die Fluoridionenquelle Zinnfluorid umfasst.

11. Mundpflegezusammensetzung nach einem vorhergehenden Anspruch, umfassend:

> a. etwa 1,0 Gew.-% Zinkoxid;
> b. etwa 0,5 Gew.-% Zinkcitrat;
> c. etwa 1,5 Gew.-% L-Arginin;
> d. 0,5 Gew.-% bis 1 Gew.-% Hydroxyethylcellulose; und

0,3 Gew.-% bis 0,8 Gew.-% Xanthan, vorzugsweise in einer Menge von 0,3 Gew.-% bis 0,6 Gew.-%, oder von 0,7 Gew.-% bis 0,8 Gew.-%.

12. Mundpflegezusammensetzung nach einem vorhergehenden Anspruch, wobei die Mundpflegezusammesetzung

> i) in einer Form vorliegt, ausgewählt aus: einer Zahnpasta, einem Mundwasser und einem Gel; und/oder
> ii) eine statische Fließgrenze von mehr als 20 Pa, 25 Pa, 30 Pa, 35 Pa oder 40 Pa aufweist.

13. Mundpflegezusammensetzung nach einem vorhergehenden Anspruch zur Verwendung in einem Verfahren zur Verbesserung der Mundgesundheit, umfassend das Auftragen einer wirksamen Menge der Mundpflegezusammensetzung in der Mundhöhle eines Subjekts, das diese benötigt, wobei das Verfahren wirksam ist, um:

> i. die Bildung von Zahnkaries zu reduzieren oder zu hemmen,
> ii. die Demineralisierung der Zähne zu reduzieren oder zu hemmen,
> iii. die Überempfindlichkeit der Zähne zu reduzieren,
> iv. Gingivitis zu reduzieren oder zu hemmen,
> v. die Heilung von Wunden oder Schnitten im Mund zu fördern,
> vi. den Gehalt an säurebildenden Bakterien zu reduzieren,
> vii. den relativen Gehalt an arginolytischen Bakterien zu erhöhen,
> viii. die Bildung mikrobieller Biofilme in der Mundhöhle zu hemmen,
> ix. die Ansammlung von Plaque zu reduzieren,
> x. die Erosion der Zähne zu reduzieren; und/oder
> xi. die Zähne und die Mundhöhle zu reinigen.

**Revendications**

1. Composition de soin buccal comprenant :

> a. un acide aminé basique sous forme libre ou de sel, l'acide aminé basique étant choisi parmi l'arginine, la lysine et une combinaison de celles-ci ;
> b. une combinaison de sources d'ions zinc ; et
> c. un système épaississant comprenant :
>
> > i. de 0,25 % en poids à 1 % en poids d'un éther de cellulose non ionique, ayant une masse moléculaire de 300 000 à 350 000 ; et

    ii. de 0,4 % en poids à 1 % en poids d'une gomme de polysaccharide.

2. Composition de soin buccal selon la revendication 1, dans laquelle l'éther de cellulose non ionique a une viscosité, mesurée à 2 % dans l'eau à 25°C, de 150 à 400 cps.

3. Composition de soin buccal selon l'une quelconque des revendications précédentes, comprenant de l'éther de cellulose non ionique dans une quantité choisie parmi 0,5% en poids à 1 % en poids, 0,6 % en poids à 0,9 % en poids, 0,5 % en poids, 0,6 % en poids, 0,7 % en poids, 0,8 % en poids, 0,9 % en poids ou 1 % en poids.

4. Composition de soin buccal selon l'une quelconque des revendications précédentes, dans laquelle l'éther de cellulose non ionique comprend de l'hydroxyéthylcellulose.

5. Composition de soin buccal selon l'une quelconque des revendications précédentes, comprenant une gomme de polysaccharide dans une quantité choisi parmi 0,4 % en poids à 0,9 % en poids, 0,5 % en poids à 0,9 % en poids, de 0,6 % en poids à 0,9 % en poids, environ 0,3 % en poids, environ 0,4 % en poids, environ 0,5 % en poids, environ 0,6 % en poids, environ 0,7 % en poids, environ 0,8 % en poids ou environ 0,9 % en poids.

6. Composition de soin buccal selon l'une quelconque des revendications précédentes, dans laquelle la gomme de polysaccharide est la gomme de xanthane.

7. Composition de soin buccal selon l'une quelconque des revendications précédentes, dans laquelle le système épaississant comprend en outre environ 5 % en poids à 10 % en poids de silice ; et / ou dans laquelle l'éther de cellulose non ionique et la gomme de polysaccharide sont présents dans un rapport en poids choisi parmi 3:1 à 1:2, 2:1 à 1:1, environ 1:1, 3:1, 2,5:1, 2:1, 1,5:1 ou 1:1.

8. Composition de soin buccal selon l'une quelconque des revendications précédentes, dans laquelle

    (i) la combinaison de sources d'ions zinc comprend de l'oxyde de zinc et du citrate de zinc, et / ou
    (ii) l'acide aminé est l'arginine et est présent à environ 1,5 % en poids, 5 % en poids, ou environ 8 % en poids de la composition de soin buccal, de préférence le rapport en poids de l'oxyde de zinc au citrate de zinc étant d'environ 2:1.

9. Composition de soin buccal selon la revendication 8, dans laquelle le citrate de zinc est dans une quantité d'environ 0,5 % en poids et l'oxyde de zinc est présent dans une quantité d'environ 1,0 % en poids sur la base du poids total de la composition de soin buccal.

10. Composition de soin buccal selon l'une quelconque des revendications précédentes, comprenant en outre une source d'ions fluorure choisie parmi le fluorure de sodium, le monofluorophosphate de sodium et le fluorure stanneux, de préférence dans laquelle la source d'ions fluorure fournit du fluorure soluble dans une quantité d'environ 1450 ppm, et / ou dans laquelle la source d'ions fluorure comprend du fluorure stanneux.

11. Composition de soin buccal selon l'une quelconque des revendications précédentes, comprenant :

    a. environ 1,0 % en poids d'oxyde de zinc ;
    b. environ 0,5 % en poids de citrate de zinc ;
    c. environ 1,5 % en poids de L-arginine ;
    d. de 0,5 % en poids à 1 % en poids d'hydroxyéthylcellulose ; et

de 0,3 % en poids à 0,8 % en poids de gomme de xanthane, de préférence dans une quantité de 0,3 % en poids à 0,6 % en poids ou de 0,7 % en poids à 0,8 % en poids.

12. Composition de soin buccal selon l'une quelconque des revendications précédentes, dans laquelle la composition de soin buccal

    i) est sous une forme choisie parmi : une pâte dentifrice, un bain de bouche et un gel ; et / ou
    ii) a une contrainte d'écoulement statique supérieure à 20 Pa, 25 Pa, 30 Pa, 35 Pa ou 40 Pa.

**13.** Composition de soin buccal selon l'une quelconque des revendications précédentes pour une utilisation dans un procédé d'amélioration de la santé buccale, comprenant l'application d'une quantité efficace de ladite composition de soin buccal à la cavité buccale d'un sujet en ayant besoin, dans laquelle le procédé est efficace pour :

> i. réduire ou inhiber la formation de caries dentaires ;
> ii. réduire ou inhiber la déminéralisation des dents ;
> iii. réduire l'hypersensibilité des dents ;
> iv. réduire ou inhiber la gingivite ;
> v. favoriser la guérison des plaies ou des coupures dans la bouche ;
> vi. réduire les taux de bactéries productrices d'acide ;
> vii. augmenter les taux relatifs de bactéries arginolytiques ;
> viii. inhiber la formation de bio films microbiens dans la cavité buccale ;
> ix. réduire l'accumulation de plaque ;
> x. réduire l'érosion des dents ; et / ou
> xi. nettoyer les dents et la cavité buccale.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3535421 A, Briner
- US 4885155 A, Parran, Jr.
- US 3678154 A, Widder
- US 4842847 A
- US 4866161 A, Sikes
- WO 2013089734 A1

**Non-patent literature cited in the description**

- **J.M. HUBER FINLAND OY.** *Telakkatie 5 FIN-49460 Hamina*